# EUROPEAN PATENT APPLICATION

(11) **EP 2 000 171 A2**
(43) Date of publication of application: **10.12.2008**
(21) Application number: 07714405.3
(22) Date of filing: 16.02.2007
(51) Int. Cl.: A61M 39/00

(54) **MEDICAL STOPCOCK**

(30) Priority: 30.03.2006 JP 2006094401
(71) Applicant: TERUMO KABUSHIKI KAISHA, Tokyo 151-0072 (JP)
(72) Inventor: KANEKO, Takashi, Fujinomiya-shi, Shizuoka 418-0015 (JP); OONISHI, Shuuichi, Fujinomiya-shi, Shizuoka 418-0015 (JP); JOUKAJI, Mitsuru, Fujinomiya-shi, Shizuoka 418-0015 (JP)
(74) Representative: Dossmann, Gérard
(86) International application number: PCT/JP2007/052875
(87) International publication number: WO 2007/116610

(57) **Abstract**

It is intended to provide a medical stopcock **characterized by** having a housing made of a polyarylene sulfide resin and a cock made of a polyolefin-based resin, which has such pressure-proofness as being usable in angiographic techniques, high chemical resistance (Lipiodol resistance) and tolerance to radiation sterilization, in particular, tolerance to electron beam sterilization.

## Description

### Technical Field

The present invention relates to medical devices, particularly to a medical stopcock. More particularly, the invention relates to a medical stopcock for used with a catheter such as an angiographic catheter.

### Background Art

A medical stopcock is an indispensable device which has the role of preventing liquid leakage, the role of changing over the liquid medicines or the like, at the time of injecting a contrast medium or a liquid medical in a medical site, particularly in a medical procedure using a catheter.

In a medical procedure using an angiographic catheter, a contrast medium having a high viscosity is injected, so that the stopcock to be used is required to have pressure-proofness and chemical resistance.
Beside, in recent years, in addition to the just-mentioned requirement, promotion of conversion to sterilization by radiation such as electron beam or γ-ray is also demanded in view of environmental issues. The conversion to radiation sterilization, particularly electron-beam sterilization is of great importance now on, in view of such environmental issues as control of emission of ethylene oxide gas (EOG), which is one of specified chemical substances.

Sterilization techniques in the related art include batch sterilization using the EOG. This method, however, has a problem in that residual EOG is left and, since it is needed to provide an aeration period before shipping, the storage period after sterilization would be long (refer to Patent Document 1). In addition, the EOG is designated as a specified chemical substance, and a vigorous management is needed in handling the EOG.

On the other hand, sterilization by radiation such as electron beams and γ-rays is advantageous in that the radiation is capable of penetration into the products and packages and, further, no residue is left. Therefore, conversion to the sterilization by radiation such as electron beams and γ-rays has been promoted. Accordingly, the stopcock is required also to have tolerance to radiation sterilization.

Conventionally, because stopcocks for use with catheters have been produced by a process in which a polycarbonate resin, a polypropylene resin or a polyoxymethylene resin is mainly used as a constituent material for a housing, whereas a polyethylene resin, a polyoxymethylene resin or a polytetrafluoroethylene (PTFE) resin is used as a constituent material for a cock for use with the housing, in consideration of the demand for high rigidity on a material basis and for comparatively good moldability at the time of molding the product.
However, the polycarbonate resin as a material for the housing is susceptible to crazing or cracking upon contact with a specified organic compound (for example, organic solvents such as acetone, oily contrast media such as iodized poppy oil ethyl ester, etc.). In addition, it has been reported that polycarbonate-made stopcocks and connectors may be broken in their use for medical procedure (refer to, for example, Non-Patent Document 1). In particular, the stopcocks and connectors are liable to be broken at joint portions on which a pressure is exerted.

In medical sites, particularly in the sites of catheter procedure, oily contrast media such as Lipiodol (registered trademark) (iodized poppy oil ethyl ester) being high in viscosity is frequently used.
Besides, in the case of dosing a patient with a carcinostatic agent, an immunosuppressor or the like, the medicine contains a solubilizing agent such as benzyl alcohol, polyoxyethylene cured castor oil, lipid emulsion, soybean oil, etc.
It is known that, when polycarbonate-made stopcocks and connectors are used for dosing a patient with such a medicine containing the solubilizing agent, the stopcocks or connectors may be broken. When the stopcock or connector is broken, not only it becomes impossible to accurately dose the patient with the medicine, but also the patient may suffer bacterial infection because the broken portion would be an open system. In addition, where a stopcock is broken during a catheter procedure, the leakage of blood cannot be stopped, and the procedure has to be stopped.
Accordingly, medical workers have suffered a heavy burden of paying close attention at the time of using such a medicine as above-mentioned.

On the other hand, polypropylene resin and polyoxyethylene resin are used as materials which are high in chemical resistance.
However, since the polypropylene resin is an aliphatic crystalline resin, it has a high shrinkage factor, lacks dimensional stability, and is insufficient in strength. Therefore, the polypropylene resin is not suitable for forming a housing of a stopcock to be applied to pressure-proof use.

In relation to the above-mentioned problem of chemical resistance, Patent Document 2 has recently proposed a medical device characterized by being formed from at least one resin selected from among polyphenyl sulfone and polyetherimide.
However, it is well-known to those skilled in the art that polyphenyl sulfone and polyetherimide, which are non-crystalline engineering plastics, are poor in precision moldability and cannot be molded with such a dimensional accuracy as to show sufficient pressure-proofness. Besides, where these materials are used to form the housings of stopcocks, they are poor in creep resistance and the pressure-proofness thereof is susceptible to deterioration with time. Therefore, these materials cannot promise sufficient pressure-proofness, and it is considered to take further time to put the products to practical use.

Patent Document 1: JP-T-2005-524804
Patent Document 2: Japanese Patent Laid-Open No. 2004-254789
Non-Patent Document 1: MASUI (Vol. 49, 2000, pp. 802-805)

### Disclosure of Invention

### Technical Problem

The above-mentioned polyoxymethylene resin has been favorably used as a material for housings and cocks of stopcocks because of its good moldability, dimensional stability, sliding properties and creep resistance.
However, as a result of the present inventors' studies on the radiation resistance of polyoxymethylene resin, it has been found that the polyoxymethylene resin exposed to radiation not only has the problem of deterioration of strength thereof but also the problem that, even if the resin is adaptable to the use on a functional basis, the resin decomposes while generating formaldehyde and/or formic acid, which essentially makes it difficult to secure safety.
The present invention has been made in consideration of the above-mentioned problems. Accordingly, it is an object of the present invention to provide a medical stopcock having pressure-proofness, chemical resistance (Lipiodol resistance) and tolerance to irradiation sterilization, particularly tolerance to electron beam sterilization.

The pressure-proofness herein means a hydrostatic pressure performance for angiographic techniques of not less than 250 psi (17 atm), more preferably not less than 500 psi (34 atm) (for hydrostatic pressure test method, refer to ISO Standard test method ISO 10555-2:1996(E), Annex A "Test for freedom from leakage and damage under pressure conditions").
In addition, the chemical resistance herein means the pressure-proofness of the medical stopcock and the presence/absence of changes in appearance such as absence of cracking, such as crazes and cracks, under visual inspection, before and after immersion in Lipiodol (registered trademark).
Further, the tolerance to radiation sterilization herein means the change in pressure-proofness upon exposure to radiation of not less than 25 kGy, which is favorably used for ordinary sterilization.

### Technical Solution

The above object is solved by the present invention residing in the following (1) to (8).
(1) A medical stopcock comprising a housing made of a polyarylene sulfide resin, and a cock made of a polyolefin-based resin.
(2) The medical stopcock as set forth in (1) above, wherein the housing made of the polyarylene sulfide resin and the cock made of the polyolefin-based resin are connected to each other by press fit.
(3) The medical stopcock as set forth in (1) or (2) above, wherein the polyarylene sulfide resin is a polyphenylene sulfide resin.
(4) The medical stopcock as set forth in any of (1) to (3) above, wherein the polyphenylene sulfide resin has an average molecular weight of 5×10³ to 5×10⁶.
(5) The medical stopcock as set forth in any of (1) to (4) above, wherein the polyphenylene sulfide resin contains at least one selected from the group consisting of glass fiber, inorganic filer, whisker and carbon fiber.
(6) The medical stopcock as set forth in any of (1) to (5) above, wherein the polyolefin-based resin is a polyethylene resin.
(7) The medical stopcock as set forth in any of (1) to (6) above, wherein said polyethylene resin has an average molecular weight of 1 × 10⁵ to 5 × 10⁶.
(8) The medical stopcock as set forth in any of (1) to (7) above, wherein the stopcock is a two-way stopcock or a three-way stopcock.

### Advantageous Effects

By utilizing the stopcock according to the present invention, it is possible to attain pressure-proofness as being usable in angiographic techniques, high chemical resistance (Lipiodol resistance) and tolerance to radiation sterilization, in particular, tolerance to electron beam sterilization.

### Brief Description of Drawings

[FIG. 1]

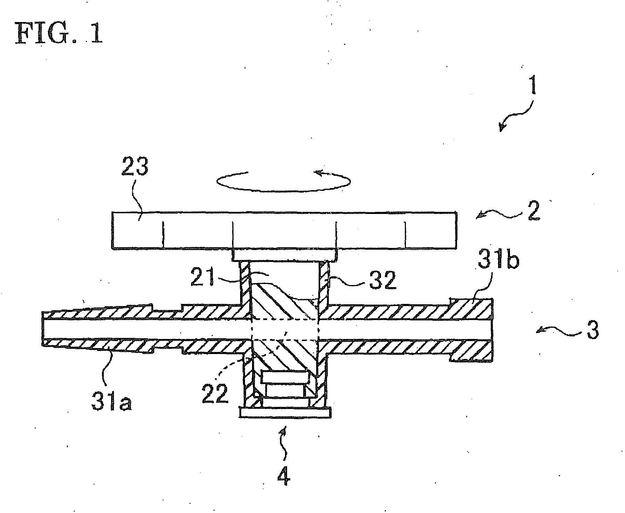

   FIG. 1 is a side view of a stopcock according to the present embodiment, which is partly sectional for understanding of the inside structure.
[FIG. 2]

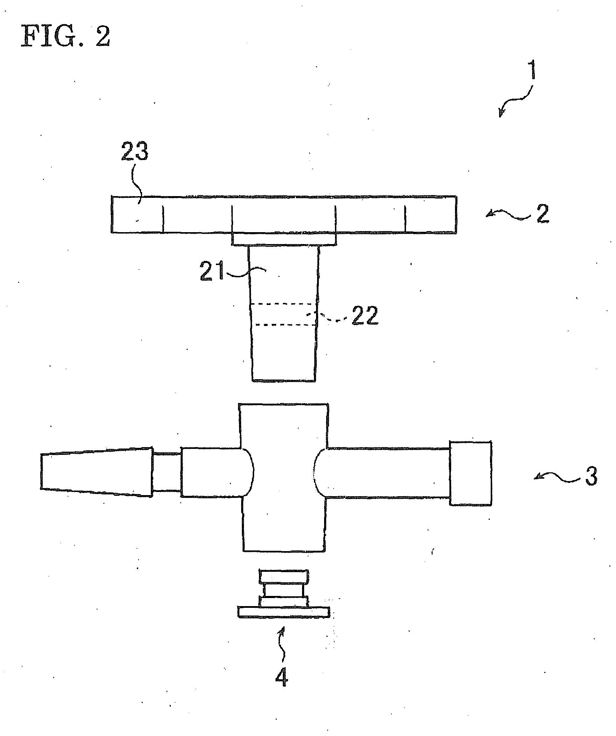

   FIG. 2 is an exploded view of the stopcock shown in FIG. 1.
[FIG. 3]

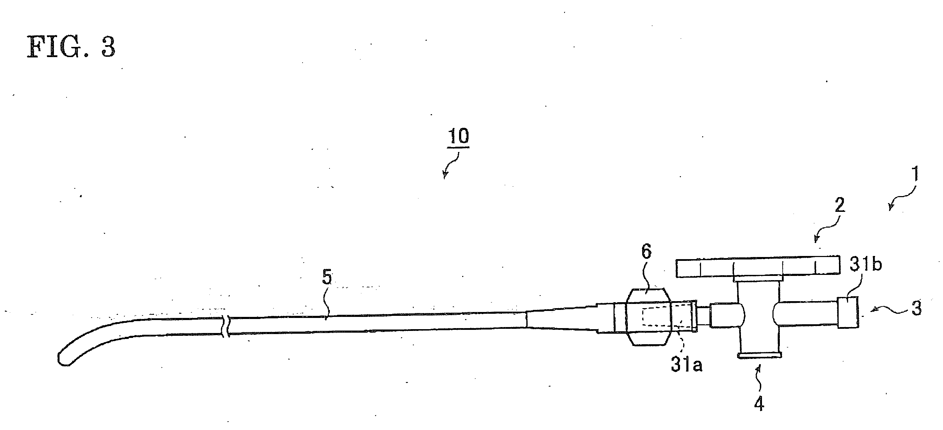

   FIG. 3 shows an assembly of the stopcock shown in FIG. 1 and a catheter.
[FIG. 4]

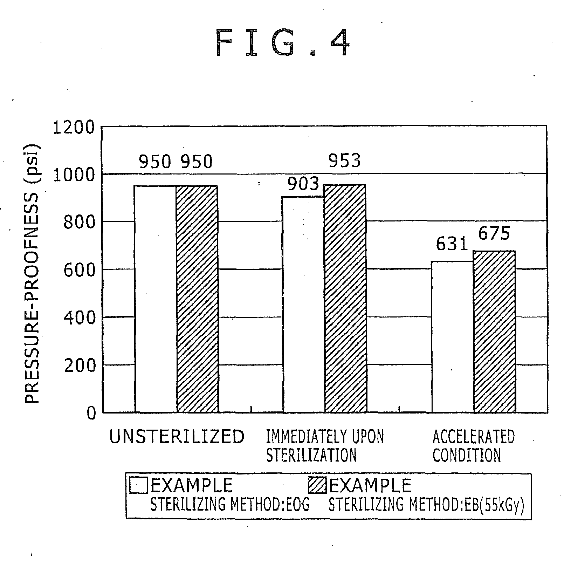

   FIG. 4 shows the results of a radiation sterilization (electron beam sterilization) tolerance test and a pressure-proofness test.

### Best Mode for Carrying Out the Invention

A stopcock according to the embodiment is connected to two or more tubes and used for opening and closing the passages of the tubes in relation to each other. Now, the stopcock according to the embodiment and the method of using the same will be described in detail below referring to the attached drawings.
FIGS. 1 and 2 show an embodiment of the stopcock according to the embodiment. FIG. 1 is a side view of the stopcock according to this embodiment, which is partly sectional for understanding of the inside structure. FIG. 2 is an exploded view of the stopcock shown in FIG. 1.
The stopcock shown in FIGS. 1 and 2 is a two-way stopcock.
As shown in FIGS. 1 and 2, the stopcock 1 in this embodiment includes a cock 2, a housing 3, and a fastener pin 4. It is to be noted that the fastener pin 4 is used in the case where fixation of the cock 2 with the fastener pin 4 is adopted; therefore, the fastener pin 4 is not indispensable, provided that the cock 2 can be fixed without any fastener.

In FIG. 1, the portion where the cock 2 and the housing 3 are combined with each other is shown in section so that the inside structure can be seen.
The stopcock 1 includes the housing 3 having a hollow cylindrical barrel part 32, and the cock 2 in which a cock body 21 having a diameter equal to or slightly greater than the diameter of an inside space of the hollow cylindrical barrel part 32 is press fitted in the inside space and which is turnably fixed by the fastener pin 4.
Press fitting is a technique by which a component part such as a cock having a comparatively larger size is fitted into a housing having a comparatively smaller size under pressure to obtain pressure-proofness, and is a technique by which a cock lower in rigidity than a rigid housing is fitted into the housing under pressure to obtain pressure-proofness.
The housing 3 has two passage ports 31a and 31b communicating with the inside space thereof, for connection to connectors of infusion tubes or the like (not shown). A handle part 23 of the cock 2 may be rotated to an ON position, to thereby cause a passage 22 formed in the cock body 21 to communicate with the passage ports 31a and 31b, or may be rotated to an OFF position, to thereby close the passage with the cock body 21.

While the medical stopcock according to the present embodiment has been described referring to the attached drawings above, the drawings merely show an example of the stopcock, and the mode for carrying out the invention is not limited to the drawings as to, for example, the number of passage ports, the shapes of the passage parts, the shape of the barrel part of the housing, the shape of the cock, etc. Thus, the stopcock may be a three-way stopcock.

Now, constituent materials of the stopcock in the present embodiment will be described.
The constituent material of the housing 3 is a polyarylene sulfide resin.
A polyarylene sulfide resin is a kind of engineering plastics. Besides, the polyarylene sulfide resin is a polymer characterized by having an aromatic compound as a recurring unit therein and having sulfur as a linking group, and is excellent in strength and rigidity, precision moldability, dimensional stability, chemical resistance, radiation resistance, and creep resistance.
Specific examples of the polyarylene sulfide resin include polyphenylene sulfide (hereinafter referred to as "PPS resin"), polythioether ketone, polythioether thioether ketone, polythioether ketone ketone, polyether thioether, polythioether sulfone, polybiphenylene sulfide, and polynaphthalene sulfide, among which particularly preferred is the PPS resin in view of its excellent radiation resistance, chemical resistance, and precision moldability and its easy availability on a commercial basis. Incidentally, the PPS resin is a crystalline aromatic engineering plastic.

The PPS resins are classified largely into a linear type, a cross-linked type, and a semi-linear type composed of a blend of the cross-linked type with the linear type.
In carrying out the present invention, any of these types of PPS resins may be used; among them, particularly preferred is the linear type PPS resin. The linear PPS resin has high strength and toughness (impact resistance) in itself, and is therefore particularly excellent.

The PPS resin has a polymerization degree of preferably 50 to 5,000, more preferably 100 to 3,000. The average molecular weight of the PPS resin is preferably 5,000 to 500,000, more preferably 10,000 to 300,000. The degree of crystallinity of the PPS resin is preferably more than 0% and not more than 60%, more preferably in the range of 5 to 40%.
With the polymerization degree, the average molecular weight and the degree of crystallinity set in the just-mentioned ranges, the PPS resin can be obtained as a material which is tough and easily moldable, is preferable as an injection molding material, and is suited to precision molding.

In the present invention, PPS as above may be used singly, alternatively, PPS may be used as a main component, which may be blended with other resin(s) or copolymerized with other resin(s) so as to be used as a PPS-based polymer alloy.
Incidentally, the polymer alloy is a concept which includes polymer blends, graft copolymers, block copolymers (micro phase separation structures) and random copolymers.

Besides, at the time of polymer alloying, alloying agents, compatibilizing agents, stabilizers, etc. may be used, as required. Examples of the other resin(s) to be polymer-alloyed with PPS include various thermoplastic resins such as modified polyolefins, fluororesins, polyethylene, polyamides, polyesters, polystyrene, polycarbonates, polyphenylene oxide, polyether ketones, polyether imides, etc., rubber components such as acrylonitrile rubber, ethylene-propylene rubber (EPR), silicone rubbers, isoprene rubber, acrylonitrile-styrene-butadiene rubber (ANSBR), etc. and so on, which may be used either singly or in combination of two or more thereof.
In addition, a blend of PPS resins differing in polymerization degree or the like may also be used. Specific examples of such a blend include blends of linear PPS with cross-liked PPS, and blends of modified PPS (meta-modified PPS) with polyparaphylene sulfide (linear PPS).
Among these resin materials, polymer blends obtained by blending PPS resins may be used, in view of their easy availability, good workability, etc.

The PPS resin as above-mentioned may contain fillers such as glass fiber, inorganic filler, whisker, carbon fiber, etc. in order to obtain rigidity preferable for use in the present invention.
Among these fillers, glass fiber and carbon fiber are preferable, and, further, glass fiber is most preferable from the viewpoints of precision molding and cost.
The content of the filler(s) added to the PPS resin is preferably 0 to 60 wt%, from the viewpoint of optimum content of fibers in a fiber-reinforced material, and is most preferably 20 to 40 wt%, from the viewpoints of moldability and commercial availability.

The PPS resin may be admixed with various additives, for improving the physical properties (mechanical properties) such as strength, rigidity, flexural elasticity, toughness, creep resistance, fatigue resistance, etc. of the finished product, for controlling the material properties such as fluidity, dimensional stability, etc. at the time of manufacture, or for improving chemical properties such as thermal resistance, chemical resistance, biocompatibility, etc. Furthermore, the PPS resin may be admixed with stabilizer, hardener, softening point regulator, pigment (colorant), etc. according to various purposes.

The constituent materials for the cock and the fastener pin are polyolefin resins.
The polyolefin resins are excellent in radiation resistance and chemical resistance, and are suitable as material of the cock which is to be press fitted into the housing made of the polyarylene sulfide resin.

Therefore, in the cock shown in FIGS. 1 and 2, it suffices that the constituent material for the cock body 21 is a polyolefin resin, and the constituent material for the handle part 23 may not necessarily be a polyolefin resin.

The polyolefin resin herein means a homopolymer or copolymer of an olefin or olefins.
Specific examples of the polyolefin resin include polyethylene, a polyethylene-polypropylene blend, an ethylene-propylene copolymer, and an ethylene-vinyl alcohol copolymer, among which preferred is polyethylene.
As the polyethylene, any of high-density polyethylene, medium-density polyethylene, and low-density polyethylene can be used, among which preferred is a high-density polyethylene of injection molding grade.

The polyethylene resin has a polymerization degree of preferably 1×10⁴ to 5×10⁵, more preferably 1×10⁴ to 3×10⁵. The average molecular weight of the polyethylene resin is preferably 1×10⁵ to 5×10⁶, more preferably 1×10⁵ to 3×10⁶.
With the above-mentioned material used and with its polymerization degree and average molecular weight set in the just-mentioned ranges, such a hardness as to promise the intended pressure-proofness and such an elasticity as to permit press fit in assembling the stopcock can be obtained.

Besides, the polyethylene resin as above may be used either singly or may be used as a main component, which may be blended with other resin(s) or copolymerized with other resin(s) so as to be used as a polyethylene-based polymer alloy, within such a range that solvent resistance of the resin material is not spoiled.
Examples of the other resin(s) include polypropylene, polybutene, and an ethylene-vinyl alcohol copolymer. Specific examples of the polyethylene-based polymer alloy include polyethylene-polypropylene blends and polyethylene-polypropylene copolymers.

The polyethylene resin may be admixed with filler or other various additives, as has been mentioned in the description of the PPS resin above, according to the purposes.

In addition, the cock made of the polyethylene resin may have cores made of other resin at other portions than the portions making contact with a liquid or with the housing. Examples of the other resin(s) here include PPS, polybutylene terephthalate (PBT), polyethylene terephthalate (PET), and polycarbonates. Besides, examples of the constituent material for the handle part include polyethylene resin, ABS resin, ethylene-propylene rubber (EPR), isoprene rubber, silicone rubbers, polystyrene resin, styreneacrylonitrile resin (SAN resin), polypropylene resin, styrene-ethylene/butylenes-styrene block copolymer (SEBS resin), and ethylene-propylene-diene terpolymer (EPDM resin).

The elements of the stopcock according to the present invention, i.e., the housing, the cock and the fastener pin can be formed by known forming methods, for example, injection molding, cutting forming, or compression molding.
The polyethylene resin-made cock having a core made of other resin can be formed by two-color molding.
The cock is press fitted in the housing obtained in the above-mentioned procedure, and the cock is fixed by use of the fastener pin, whereby the stopcock 1 shown in FIGS. 1 and 2 is obtained.

The stopcock according to the present invention, wherein the cock made of the polyolefin resin is press fitted in the housing made of the polyarylene sulfide resin, has such pressure-proofness and chemical resistance (Lipiodol resistance) as being usable in angiographic catheterization and tolerance to radiation sterilization, in particular, tolerance to electron beam sterilization. In addition, from the results of Examples which will be described later, it is seen that the stopcock according to the present invention is free from lowering in pressure-proofness upon radiation sterilization and, moreover, can be expected to be slightly enhanced in pressure-proofness upon sterilization.

FIG. 3 is a drawing for illustrating a use mode of the stopcock, and shows an assembly of a catheter and the stopcock. In the assembly 10 shown in FIG. 3, the passage port 31a in the stopcock 1 shown in FIGS. 1 and 2 and a hub 6 provided on the proximal end side of a catheter (angiographic catheter) 5 are connected to each other by use of a lock adapter (not shown). The lock adapter may be present or absent, but it is preferable to use the lock adapter in order to further strengthen the hub 6 and the passage port 31a.

In use of the assembly 10 shown in FIG. 3, in the condition where the distal end side of the catheter (angiographic catheter) 5 is placed in a blood vessel, a pressure-proof extension tube (not shown) extending from a mechanical injection device (referred to also as injector; not shown) is connected liquid-tight to the passage port 31b of the stopcock 1 by utilizing a lure taper, and a liquid medicine prepared by mixing Lipiodol (registered trademark) with a carcinostatic agent or the like is injected from the injector. Or, alternatively, a syringe (not shown) is connected to the passage port 31b through a pressure-proof extension tube, and a liquid medicine is manually injected while viewing a monitor.

While the stopcock 1 has to have chemical resistance and pressure-proofness because an oily contrast medium having a high viscosity is injected therethrough, the stopcock in the present embodiment has sufficient chemical resistance and pressure-proofness. At the time of injecting the contrast medium, the cock 2 of the stopcock 1 is in the ON position, permitting a liquid to pass therethrough. During an operation of the catheter 5, the cock 2 is put in the OFF position, so that the operator can freely operate the catheter 5 without being restricted in any way by the extension tube or the syringe, and there is no fear of leakage of blood.

Incidentally, sterilization of the stopcock 1 can be performed by utilizing EOG sterilization, hydrogen peroxide sterilization, or other sterilizing methods, but it is preferable to perform the sterilization by radiation sterilization using γ-rays or electron beams, in particular, electron beam sterilization. Besides, any of almost all the sterilization methods ordinarily used in medical sites, such as alcohol sterilization, ozone sterilization, UV sterilization, etc. can be used.
Therefore, the stopcock 1 sterilized without using EOG sterilization can be favorably used without fear of hemolytic toxicity due to residual EOG or the like.

### Example

Now, the present invention will be described more in detail below by way of Example, which is not limitative of the invention.

### <Example>

In the following example, the two-way stopcock 1 shown in FIGS. 1 and 2 was used.

### (1) Molding of Housing

The housing 3 was obtained by injection molding of a linear PPS resin (FORTRON (registered trademark) PPS, grade 1130A6, produced by Polyplastics, Co., Ltd.) containing 30 wt% of glass fibers, under the conditions recommended by the manufacturer. With respect to each of the resins used in Comparative Examples, also, an injection molded body was obtained by injection molding under the recommended conditions of the relevant resin.

### (2) Molding of Cock and Fastener Pin

The cock 2 having the cock body 21 and the handle part 23 was obtained by injection molding conducted by using an injection grade of high-density polyethylene resin (grade HJ560, produced by Japan Polyethylene Corporation) under the maker-recommended conditions. Also, the fastener pin was similarly obtained by injection molding of a polyethylene resin (grade HJ490, produced by Japan Polyethylene Corporation). With regard to each of the resins used in Comparative Examples, also, an injection molded body was obtained by injection molding under the recommended conditions of the relevant resin.

### (3) Assemblage of Stopcock and Connection with Catheter

A silicone oil was applied to the outer periphery of the cock body 21, the cock body 21 is press fitted into the inside space of the hollow cylindrical barrel part 32 of the housing 3, and then the cock body 21 was fixed by the fastener pin 4, to assemble the stopcock as shown in FIG. 1. The passage port 31a of the stopcock 1 was connected to the hub 6 of a catheter 5 by use of lure taper fitting and an adhesive, and was fixed by a lock adapter (not shown), to obtain the assembly 10 shown in FIG. 3.

### (4) Radiation Sterilization (Electron Beam Sterilization) and EOG Sterilization

The assemblies 10 obtained following the above-mentioned procedure were each individually packaged with Tyvek (registered trademark), which is a gas-permeable nonwoven fabric packaging material, and were subjected to radiation sterilization (electron beam sterilization) at an exposure dose of 33 or 55 kGy.
Normally, the exposure dose at the time of electron beam sterilization is 25 kGy, but, in the case of the radiation sterilization (electron beam sterilization), an actual exposure and a maximum allowable exposure radiation are desired, taking the exposure distribution into consideration. Therefore, evaluation was made under such a condition that material deterioration is more liable to occur than usual.
After the sterilization, the stopcock was subjected to the following pressure-proof test (hydrostatic pressure test) and cock slidability test, the following chemical resistance (Lipiodol resistance [oily contrast medium resistance]) test and cock slidability test, and to various safety tests. Incidentally, for comparison, EOG sterilization used ordinarily was also conducted.
The EOG sterilization was carried out under the conditions of a temperature of 40 to 70°C, a humidity of 40 to 100%, an EOG concentration of 450 to 850 mg/L, and an action time of 2 to 8 hr.

### (5) Pressure-proof Test (Hydrostatic Pressure Test) and Cock Slidability Test

The assemblies 10 obtained in the above-mentioned procedure were subjected to radiation sterilization (electron beam sterilization) at an exposure dose of 33 or 55 kGy, and then subjected to a pressure-proof test (hydrostatic pressure test) according to the ISO Standard test method (ISO 10555-2:1996 (E), Annex A "Test for freedom from leakage and damage under pressure conditions"). Similarly, the assemblies 10 having been left to stand in an oven at 60°C for one week (accelerated condition) after sterilization were also subjected to the pressure-proof test.
Here, the test was conducted at an environmental temperature of 37°C, using service water as a pressure medium. This means severer conditions, with regard to leakage, than those in the case of a viscous contrast medium. Besides, holding at 60°C for seven days is considered to correspond to holding under room temperature condition for 180 days (Japanese Standard Association, ISO Standards, translated edition "Sterilization method/sterilization validation/sterilization assurance for medical appliances" (1996, 1st edition, 1st print), p. 74, ISO Standard 11137 Annex A (reference) Performance verification of appliances and packaging materials).
The pressure-proofness performance (hydrostatic pressure performance) is preferably not less than 250 psi, and more preferably not less than 500 psi.
The results of these pressure-proof tests are shown in Tables 1 and 2.

In addition, the assemblies 10 obtained following the above-mentioned procedure were subjected also to a cock slidability test, after each sterilization. With regard to cock operation, the maximum torque (kgf· cm) generated when the cock was rotated was measured by use of a commercially available torque gauge. The cock slidability, in terms of the maximum torque, is preferably not more than 3.0 kgf· cm, and more preferably not more than 2.0 kgf· cm.
The results of the cock slidability tests are shown in Tables 3 and 4.

### (6) Chemical Resistance (Lipiodol Resistance) Test and Cock Slidability Test

The assemblies 10 obtained in the above-mentioned procedure were filled with Lipiodol (registered trademark), and left to stand at room temperature, and a cock slidability test and a pressure-proof test were repeated with the lapse of time.
The filling was carried out by a method in which the cock 2 in the stopcock 1 was put into the state of permitting a liquid to flow therethrough, a glass-made syringe (not shown) was connected to the side of the passage port 31b, Lipiodol (registered trademark) was injected, and, after confirming flowing-out of Lipiodol from the distal end opening of the catheter 5, the tube of the catheter 5 was clamped with metallic forceps (not shown), then the glass-made syringe was detached, and a cap was attached to the passage port 31b.
The results were evaluated in terms of the cock operation measured value (the maximum torque [kgf· cm] generated at the time of rotation of the cock was measured by use of a commercially available torque gauge), the pressure-proof test and the presence/absence of a change in appearance.
The results are shown in Table 5.

### (7) Various Safety Tests

In consideration of safety of medical appliances, the assemblies 10 obtained in the above-mentioned procedure were subjected to an eluate test (pH/potassium permanganate reducing substance) and a cell toxicity test according to the plastic-made medicine vessel test methods described in the 14th Revised Japanese Pharmacopoeia.
It is to be noted here that the stopcocks subjected to these tests were produced to be higher in accuracy than the stopcocks subjected to tests in (5) and (6) above.
The results of the eluate test were evaluated in terms of whether or not the safety was fulfilled, according to the dialytic artificial kidney system approval standards. Specifically, it was evaluated that safety was fulfillled in the case where the difference in pH between the result of the pH test in the eluate test for the tested liquid and that for a control test liquid was less than 1.5 and where the difference in potassium permanganate solution consumption between the result of the potassium permanganate reducing substance test for a tested liquid and that for a control test liquid was not more than 1.0 ml.
The results of the cell toxicity test were evaluated according to YAKKI, No. 91 "Guidelines for fundamental biological tests of medical appliappliancesances and medical materials." Specifically, the colony forming activity of a fresh culture medium was compared with those of eluates diluted stepwise to various concentrations, and the intensity of cell toxicity in the eluate was represented by such an eluate concentration (IC₅₀) as to inhibit by 50% the number of colonies in the fresh culture medium. Then, the specimens giving an IC₅₀ value of not less than 90% were evaluated as being on an acceptable level.
Together with the safety tests, a cock slidability test, a pressure-proof test and a chemical resistance (Lipiodol resistance) test were also conducted according to the above-mentioned methods, for evaluation of the stopcocks. The results of the pressure-proof test and the chemical resistance (Lipiodol resistance) test are shown in Table 6.

### (8) Radiation Sterilization (Electron Beam Sterilization) Test and Pressure-proof Test

The assemblies 10 obtained in the above-mentioned procedure were subjected to a pressure-proof test in the same procedure as in (5) above, in order to examine the influences of the constituent materials.
The test results are shown in FIG. 4.

### [Comparative Examples]

A polyoxymethylene (POM) resin (polyacetal resin) and a polyarylate (PAR) resin were used as the constituent material for the housing 3, for comparison with the linear PPS resin (containing 30 wt% of glass fibers).
For comparison with Patent Document 2, the present inventors hoped to select a polyphenylsulfone resin and a polyether imide resin, but they abandoned the hope because these resins were so high in molding temperature that they were difficult to mold by ordinarily used molding machines and that no acceptable molded product could easily be obtained. As an alternative, a polyarylate resin was adopted as a representative of non-crystalline aromatic engineering plastics.
In addition, as the constituent material for the cock 2, polyoxymethylene resins were used other than the high-density polyethylene resin, for comparison.

### <Comparative Example 1>

A stopcock 1 was produced in the same manner as in Example, except that the constituent material for the cock 2 was changed to a polyoxymethylene resin (grade SF-20/impact-resistant flexible grade, produced by Polyplastics Co., Ltd.).

### <Comparative Example 2>

A stopcock 1 was produced in the same manner as in Example, except that the constituent material for the housing 3 was changed to a polycarbonate resin (grade S1001R, produced by Mitsubishi Engineering-Plastics Corporation).

### <Comparative Example 3>

A stopcock 1 was produced in the same manner as in Example, except that the constituent material for the housing 3 was changed to a polycarbonate resin (grade S1001R, produced by Mitsubishi Engineering-Plastics Corporation) and the constituent material for the cock 2 was changed to a polyoxymethylene resin (grade SF-20/impact-resistant flexible grade, produced by Polyplastics Co., Ltd.).

### <Comparative Example 4>

A stopcock 1 was produced in the same manner as in Example, except that the constituent material for the housing 3 was changed to a polyarylate (PAR) resin (grade U-8000, produced by Unitika Ltd.).

### <Comparative Example 5>

A stopcock 1 was produced in the same manner as in Example, except that the constituent material for the housing 3 was changed to a polyarylate (PAR) resin (grade U-8000, produced by Unitika Ltd.) and the constituent material for the cock 2 was changed to a polyoxymethylene resin (grade SF-20/impact-resistant flexible grade, produced by Polyplastics Co., Ltd.).

### <Comparative Example 6>

A stopcock 1 was produced in the same manner as in Example, except that the constituent material for the housing 3 was changed to a polyoxymethylene resin (grade M-270, produced by Polyplastics Co., Ltd.) and the constituent material for the cock 2 was changed to a polyoxymethylene resin (grade YF-20/slidable grade, produced by Polyplastics Co., Ltd.).

<Results of Pressure-proof Test (Hydrostatic Pressure Test) and Cock Slidability Test>

**[Table 1]**

| Results of pressure-proof test (psi), Immediately upon sterilization | | | | | | |
|---|---|---|---|---|---|---|
| | Combination of Constituent materials | | Unsterilized | EOG Sterilization | EB sterilization 33 kGy | EB sterilization 55 kGy |
| | Housing | Cock | | | | |
| Example | PPS-GF30 | HDPE | 1008 | 852 | 967 | 865 |
| Comp.Ex.1 | PPS-GF30 | POM(1) | 1003 | 823 | 898 | 809 |
| Comp.Ex.2 | PC | HDPE | 678 | 560 | 620 | 619 |
| Comp.Ex.3 | PC | POM(1) | 951 | 774 | 845 | 778 |
| Comp.Ex.4 | PAR | HDPE | 674 | 626 | 525 | 491 |
| Comp.Ex.5 | PAR | POM(1) | 903 | 682 | 802 | 718 |
| Comp.Ex.6 | POM(2) | POM(3) | 802 | 938 | 811 | 598 |

**[Table 2]**

| Results of pressure-proof test (psi), Accelerated conditions | | | | | | |
|---|---|---|---|---|---|---|
| | Combination of Constituent materials | | Unsterilized | EOG Sterilization | EB sterilization 33 kGy | EB sterilization 55 kGy |
| | Housing | Cock | | | | |
| Example | PPS-GF30 | HDPE | 835 | 687 | 797 | 700 |
| Comp.Ex.1 | PPS-GF30 | POM(1) | 843 | 662 | 735 | 634 |
| Comp.Ex.2 | PC | HDPE | 508 | 390 | 460 | 454 |
| Comp.Ex.3 | PC | POM(1) | 780 | 612 | 675 | 618 |
| Comp.Ex.4 | PAR | HDPE | 514 | 466 | 375 | 341 |
| Comp.Ex.5 | PAR | POM(1) | 743 | 572 | 592 | 542 |
| Comp.Ex.6 | POM(2) | POM(3) | 732 | 713 | 581 | 7 |

**[Table 3]**

| Results of cock slidability test (kgf· cm), Immediately upon sterilization | | | | | | |
|---|---|---|---|---|---|---|
| | Combination of Constituent materials | | Unsterilized | EOG Sterilization | EB sterilization 33 kGy | EB sterilization 55 kGy |
| | Housing | Cock | | | | |
| Example | PPS-GF30 | HDPE | 1.3 | 1.4 | 1.3 | 1.4 |
| Comp.Ex.1 | PPS-GF30 | POM(1) | 2.3 | 2.0 | 2.0 | 1.8 |
| Comp.Ex.2 | PC | HDPE | 1.1 | 0.9 | 1.1 | 1.3 |
| Comp.Ex.3 | PC | POM(1) | 1.0 | 1.0 | 1.9 | 1.6 |
| Comp.Ex.4 | PAR | HDPE | 1.4 | 1.2 | 1.6 | 1.4 |
| Comp.Ex.5 | PAR | POM(1) | 1.6 | 1.5 | 1.8 | 1.6 |
| Comp.Ex.6 | POM(2) | POM(3) | 0.8 | 1.2 | 1.5 | 1.1 |

**[Table 4]**

| Results of cock slidability test (kgf· cm), Accelerated conditions | | | | | | |
|---|---|---|---|---|---|---|
| | Combination of Constituent materials | | Unsterilized | EOG Sterilization | EB sterilization 33 kGy | EB sterilization 55 kGy |
| | Housing | Cock | | | | |
| Example | PPS-GF30 | HDPE | 1.0 | 1.1 | 1.3 | 1.2 |
| Comp.Ex.1 | PPS-GF30 | POM(1) | 2.1 | 2.0 | 1.8 | 1.8 |
| Comp.Ex.2 | PC | HDPE | 1.0 | 1.0 | 0.9 | 0.8 |
| Comp.Ex.3 | PC | POM(1) | 2.1 | 1.8 | 1.8 | 1.8 |
| Comp.Ex.4 | PAR | HDPE | 1.2 | 1.0 | 1.0 | 1.0 |
| Comp.Ex.5 | PAR | POM(1) | 1.6 | 1.5 | 1.3 | 1.2 |
| Comp.Ex.6 | POM(2) | POM(3) | 1.2 | 1.2 | 1.4 | 0.9 |

The abbreviations of the resin names in the tables above are as follows.
PPS-GF30: Polyphenylene sulfide resin containing 30 wt% of glass fibers
HDPE: High-density polyethylene resin
POM(1): Polyoxymethylene resin, impact-resistant flexible grade SF-20, produced by Polyplastics Co., Ltd.
POM(2): Polyoxymethylene resin, grade M270, produced by Polyplastics Co., Ltd.
POM(3): Polyoxymethylene resin, slidable grade YF-20, produced by Polyplastics Co., Ltd.
PAR: Polyarylate resin
PC: Polycarbonate resin
EOG: Ethylene oxide gas
EB: Electron beam

From Table 1, it is clearly seen that in the unsterilized condition and in the condition immediately after EOG or EB sterilization, any combination of housing and cock will substantially fulfill the desired pressure-proofness of about 500 psi. As shown in Table 2, however, the specimens treated in accelerated conditions (left to stand in oven at 60°C for one week after radiation sterilization (electron beam sterilization)) showed clear differences in performance according to the combination of materials. Specifically, the stopcocks using a combination of a housing made of PPS-GF30 and a cock made of HDPE or POM(1) were good in pressure-proofness. On the other hand, where the housing was made of the PAR resin, which is a non-crystalline aromatic engineering plastic, it was obviously difficult to maintain the pressure-proofness of the assembly 10.
It is seen from Table 3 and 4 that any combination of housing and cock can ensure slidability, the target of which was set at a value of not more than 3.0 kgf· cm.

<Results of Chemical Resistance (Lipiodol Resistance) Test and Cock Slidability Test>

**[Table 5]**

| | Combination of Constituent materials | | Immediately upon assemblage | 1 hr after filling | 2 days after filling | Comment, observation of appearance |
|---|---|---|---|---|---|---|
| | Housing | Cock | | | | |
| Example | PPS-GF30 | HDPE | 860 | 1013 | 950 | No change, Pressure- proofness in dispersion range |
| Comp.Ex.2 | PC | HDPE | 979 | - | - | Holder crack, 10 min after filling |
| Comp.Ex.4 | PAR | HDPE | 909 | 858 | - | Holder crack, after left to stand for 24 hr |

| | | | | | | |
|---|---|---|---|---|---|---|
| Unit: psi | | | | | | |

From the results of the Lipiodol resistance test shown in Table 5, it is clear that the PPS resin-made housing had good Lipiodol resistance, and can ensure a target vale of cock slidability (results being not shown) of not more than 3.0 kgf· cm. On the other hand, the PC resin-made housing and PAR resin-made housing underwent environmental stress cracking (ESC), so that the test could not be continued, indicating that they are insufficient in Lipiodol resistance.
From these results, it is considered that the aromatic non-crystalline engineering plastics such as the PAR resin are insufficient in creep resistance and ESC resistance, as compared to the aromatic crystalline engineering plastics, and are not suited to use for forming the housings of the stopcocks. Besides, they are not necessarily good in moldability.

<Results of Various Safety Tests>

**[Table 6]**

| Subject matter of investigation | Example | Comp. Ex. 1 |
|---|---|---|
| Combination of constituent materials | Housing: PPS-GF30, Cock: HDPE | Housing: PPS-GF30, Cock: POM(1) |
| Cock slidability test (unsterilized, sliding torque) | 1.1 kgf· cm | 1.1 kgf· cm |
| Cock slidability test (after EB 55 kGy sterilization) | 0.34 kgf· cm | 1.12 kgf· cm |
| Pressure-proof test (unsterilized) | 950 psi | 1085 psi |
| Pressure-proof test (immediately after EB 55 kGy sterilization) | 953 psi | 1070 psi |
| Pressure-proof test (EB 55 kGy sterilization, accelerated conditions) | 675 psi | 546 psi |
| Eluate test (pH) (EB 55 kGy sterilized product, ΔpH value) | 0.34 | 1.12 |
| Eluate test (potassium permanganate reducing substance) (EB 55 kGy sterilized product, ΔKMnO₄ value) | 0.05 mL | 0.9 mL |
| Cell toxicity test (IC₅₀) (EB 55 kGy sterilized product, IC₅₀) | 100% | 25%< |
| Chemical resistance (Lipiodol resistance) Test (immediately upon EB 55 kGy sterilization) | 900 psi | 830 psi |

From the results shown in Tables 1 to 5, it was considered that the preferable combinations of materials for medical stopcocks are those having a PPS resin housing and a HDPE cock or POM cock.
As shown in Table 6, however, the POM resin was acceptable as to the pressure-proof test, the cock slidability test and the chemical resistance (Lipiodol resistance) test, but was rejectable in regard of the Japanese Pharmacopoeia or the biological guideline test, adopted as a standard relating to the cell toxicity test. The object of the present invention is to provide a medical appliance needed in practical medical sites, therefore, any material that will possibly produce a safety problem cannot be adopted.

### <Results of Radiation Sterilization (Electron Beam Sterilization) Tolerance Test and Pressure-proof Test>

FIG. 4 shows the results of the pressure-proof test using the combination of housing and cock materials in Example. It has been identified that the specimens subjected to electron beam sterilization have better pressure-proofness compared with the specimens subjected to EOG sterilization. Although the electron beam sterilization is known to be severer than the EOG sterilization in influence on the material in general, the good results above are considered to be obtained due to the good radiation resistance of the polyphenylene sulfide resin and the enhancement of strength of the high-density polyethylene resin (used for the cock) by electron beam cross-linking.
It is evident also from the fact that the pressure-proofness was not lowered upon electron beam sterilization and, moreover, the pressure-proofness was slightly enhanced upon the sterilization.

Therefore, from the results shown in Tables 1 to 6 and FIG. 4, it is evident that the stopcocks based on the combination of a housing made of the polyphenylene sulfide resin (PPS-GF30) and a cock made of the polyethylene resin (HDPE) have such pressure-proofness as being usable in angiographic techniques and have chemical resistance (Lipiodol resistance) and tolerance to radiation sterilization, in particular, tolerance to electron beam sterilization.

## Claims

1. A medical stopcock comprising a housing made of a polyarylene sulfide resin, and a cock made of a polyolefin-based resin.

2. The medical stopcock according to claim 1, wherein said housing made of the polyarylene sulfide resin and said cock made of the polyolefin-based resin are connected to each other by press fit.

3. The medical stopcock according to claim 1 or 2, wherein said polyarylene sulfide resin is a polyphenylene sulfide resin.

4. The medical stopcock according to any of claims 1 to 3, wherein said polyphenylene sulfide resin has an average molecular weight of 5×10³ to 5×10⁵.

5. The medical stopcock according to any of claims 1 to 4, wherein said polyphenylene sulfide resin contains at least one selected from the group consisting of glass fiber, inorganic filer, whisker and carbon fiber.

6. The medical stopcock according to any of claims 1 to 5, wherein said polyolefin-based resin is a polyethylene resin.

7. The medical stopcock according to any of claims 1 to 6, wherein said polyethylene resin has an average molecular weight of 1×10⁵ to 5×10⁶.

8. The medical stopcock according to any of claims 1 to 7, wherein said stopcock is a two-way stopcock or a three-way stopcock.
